(19) Europäisches Patentamt
European Patent Office
Office européen des brevets

(11) Publication number: **0 117 260**
**B1**

(12) **EUROPEAN PATENT SPECIFICATION**

(45) Date of publication of patent specification: **27.08.86**

(51) Int. Cl.$^4$: **C 07 C 103/48,** A 61 K 31/22

(21) Application number: **83101814.8**

(22) Date of filing: **24.02.83**

(54) **4-Aminobutyric acid derivatives, a process for the preparation thereof and a pharmaceutical composition containing said derivatives.**

(43) Date of publication of application:
**05.09.84 Bulletin 84/36**

(45) Publication of the grant of the patent:
**27.08.86 Bulletin 86/35**

(84) Designated Contracting States:
**CH DE FR GB IT LI NL**

(56) References cited:
**FR-A-2 111 758**
**GB-A- 552 641**
**US-A-2 519 462**
**US-A-2 557 284**

**MERCK INDEX, 10th Edition, no. 4640**

**The file contains technical information submitted after the application was filed and not included in this specification**

(73) Proprietor: **Tanabe Seiyaku Co., Ltd.**
**No. 21 Dosho-machi 3-chome Higashi-ku**
**Osaka-shi Osaka-fu (JP)**

(72) Inventor: **Yoneda, Naoto**
**A-409, No. 34, Senrioka-Naka**
**Suita-shi Osaka-fu (JP)**
Inventor: **Hayashi, Kimiaki**
**G-104, No. 27, Shin-Ashiya-Kami**
**Suita-shi Osaka-fu (JP)**
Inventor: **Sugawara, Yoichi**
**No. 377-45 Koizumi**
**Ageo-shi Saitama-ken (JP)**
Inventor: **Harigaya, Shoichi**
**No. 6-4, Terao-dai 1-chome Tama-ku**
**Kawasaki-shi Kanagawa-ken (JP)**

(74) Representative: **Hansen, Bernd, Dr.rer.nat. et al**
**Hoffmann, Eitle & Partner Patentanwälte**
**Arabellastrasse 4**
**D-8000 München 81 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 117 260 B1**

**Description**

The present invention relates to novel 4-aminobutyric acid derivatives, a process for the preparation thereof and a pharmaceutical composition containing said derivatives. More particularly, it relates to 4-aminobutyric acid derivatives of the formula:

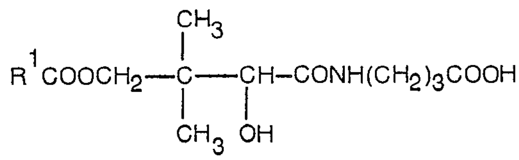

(I)

wherein $R^1$ is a straight or branched alkyl group having 1 to 8 carbon atoms, or pharmaceutically acceptable salts thereof, and a process for the preparation thereof.

It is known that calcium hopanthenate [chemical name: D-(+)-(2,4-dihydroxy-3,3-dimethylbutyramido)butyrate] relieves various symptoms such as hyperactivity, short attention span, speech disorders, hypobulia, etc. which accompany with mild mental retardation, postencephalitic syndrome, cerebral palsy, etc. and hence is useful as a remedy for amelioration of cerebral metabolism and higher brain function disorder (Merck Index, tenth edition, No. 4640).

Based on the knowledge that when calcium hopanthenate is administered orally, it is present in vivo (e.g. in blood plasma and brain) in the form of free hopanthenic acid, the present inventors have investigated various analogous compounds. As a result, it has been found that the novel compounds of the above formula (I) when administered orally are well absorbed and show higher level of bioavailability or longer duration of action in blood and brain as compared with calcium hopanthenate. Hence, they may be useful as a medicine for amelioration of cerebral metabolism and higher brain function disorder.

Specific examples of the present compounds are the compounds of the formula (I) wherein $R^1$ is a straight or branched alkyl group having 1 to 8 carbon atoms, such as methyl, ethyl, n-propyl, isopropyl, n-butyl, isobutyl, sec.-butyl, tert.-butyl, n-pentyl, isopentyl, sec.-pentyl, tert.-pentyl, 2,2-dimethylpropyl, 1-ethylpropyl, n-hexyl, n-heptyl, n-octyl, or the like. Suitable examples are the compounds of the formula (I) wherein $R^1$ is a branched alkyl group having 3 to 5 carbon atoms, such as isopropyl, isobutyl, isopentyl, tert.-butyl, 2,2-dimethylpropyl, or 1-ethylpropyl group. Particularly preferable compounds are D-4-[N-(4-isobutyryloxy-3,3-dimethyl-2-hydroxy-n-butyryl)amino]-n-butyric acid and D-4-[N-(4-isovaleroyloxy-3,3-dimethyl-2-hydroxy-n-butyryl)amino]-n-butyric acid. These compounds can favorably be obtained in the crystalline form.

The compounds (I) of the present invention can be prepared by the following processes:

A) comprising subjecting a 4-aminobutyric acid ester of the formula:

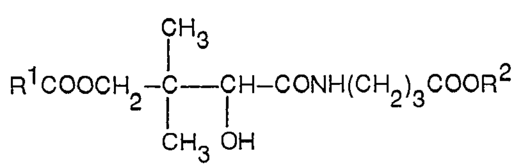

(II)

wherein $R^1$ is as defined above, and $R^2$ is a group removable by a catalytic reduction (e.g. benzyl, p-methoxybenzyl, p-chlorobenzyl, or p-nitrobenzyl group), to a catalytic reduction, or

B) comprising reacting a 4-aminobutyric acid derivative of the formula:

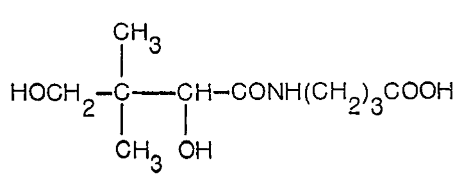

(III)

with a reactive derivative of a carboxylic acid of the formula:

$$R^1COOH \quad (IV)$$

wherein $R^1$ is as defined above.

The procedure of the above processes is explained in more detail below.

(Process A)

The starting compound (II) is catalytically reduced in an appropriate solvent in the presence of a catalyst while introducing hydrogen gas into the reaction system. The catalyst includes, for example, palladium black, palladium-carbon. The solvent includes, for example, lower alkanols (e.g. methanol, ethanol, n-propanol, isopropanol), tetrahydrofuran, dioxane, or a mixture of these solvents with water. The above reaction is usually carried out at a temperature of 0 to 60°C under 1 to 10 atm., preferably at a temperature of 10 to 30°C under 1 to 3 atm.

(Process B)

The starting compound (III) and reactive derivative of the compound (IV) are reacted in an appropriate solvent in the presence of a base. The compound (III) may be used in the form of a salt (e.g. calcium salt thereof). The reactive derivative of the compound (IV) includes any conventional derivative, preferably an acid anhydride or acid halide thereof. The base includes, for example, alkali metal hydroxide (e.g. sodium hydroxide, potassium hydroxide), alkali metal carbonates (e.g. sodium carbonate, potassium carbonate), alkali metal bicarbonates (e.g. sodium bicarbonate, potassium bicarbonate), organic tertiary amines (e.g. triethylamine, pyridine, dimethylaniline, p-dimethylaminopyridine), or the like. The solvent includes, for example, tetrahydrofuran, dioxane, chloroform, methylene chloride, benzene, ethyl acetate, dimethylformamide, diethyl ether, water, or the like, which may be used alone or in mixture thereof. The above reaction is usually carried out at a temperature of −15 to 50°C, preferably 0 to 20°C.

The compounds (I) of the present invention contain one asymmetric carbon within the molecule and hence include two optical isomers. The present invention includes also these isomers. Among these isomers, the compounds wherein the asymmetric carbon has D-configuration are particularly preferable for medical use.

The starting compound (II) used in the above process is also novel and can be prepared by the following process.

A 4-aminobutyric acid ester of the formula:

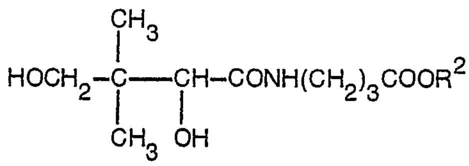

(V)

wherein $R^2$ is as defined above, is reacted with a reactive derivative of a carboxylic acid of the formula:

$$R^1COOH \qquad (IV)$$

wherein $R^1$ is as defined above.

The reaction of the compound (V) and the reactive derivative of a carboxylic acid (IV) is preferably carried out in an appropriate solvent (e.g. tetrahydrofuran, dioxane, chloroform, methylene chloride, benzene, ethyl acetate, dimethylformamide, diethyl ether, methyl ethyl ether, water, or a mixture thereof) in the presence or absence of a base (e.g. organic tertiary amines such as triethylamine, pyridine, dimethylaniline, or p-dimethylaminopyridine; or inorganic bases such as sodium hydroxide, potassium hydroxide, sodium carbonate, potassium carbonate, sodium bicarbonate, or potassium bicarbonate) at a temperature of −15 to 50°C.

The compound (V) can be prepared by reacting a metal salt or organic amine salt of 4-[N-(2,4-dihydroxy-3,3-dimethyl-n-butyryl)amino]-n-butyric acid with a compound of the formula: $R^2X$ (wherein $R^2$ is as defined above, and X is a halogen atom, such as chlorine, bromine or iodine atom), or by reacting a 4-amino-n-butyric acid ester of the formula:

$$H_2N—(CH_2)_3COOR^2 \qquad (VI)$$

wherein $R^2$ is as defined above, with a γ-lactone of 2,4-dihydroxy-3,3-dimethyl-n-butyric acid.

The compounds (I) of the present invention may be used in the form of a free acid or in the form of a pharmaceutically acceptable salt thereof. Suitable examples of the salt are, for example, calcium salt, sodium salt, potassium salt, lithium salt, magnesium salt, lysine salt, ornithine salt, or arginine salt.

The compounds (I) or a pharmaceutically acceptable salt thereof is preferably administered in oral route (but may also be administered in parenteral route) in a conventional preparation, for example, solid preparations such as tablets, pills, powders, capsules, or granules, and liquid preparations such as solutions, suspensions, or emulsions. Such preparations can be prepared in a conventional manner, for example, by admixing a compound of the formula (I) or a pharmaceutically acceptable salt thereof with a conventional carrier or diluent, such as calcium carbonate, calcium phosphate, corn starch, potato starch, lactose, talc, and magnesium stearate.

Dose of the compounds (I) or a pharmaceutically acceptable salt thereof may vary in accordance with kinds of diseases to be treated, ages and weights of patients, severity of diseases and the administration routes, but is usually in the range of 1 to 20 mg/kg/day, preferably 2 to 10 mg/kg/day, for oral administration.

When the compounds (I) or a pharmaceutically acceptable salt thereof of the present invention is administered, they show a high level in blood and brain, which were confirmed by the following experiments in vivo.

Experiment 1

SD-male rats, weighing about 200 g, 7 weeks age, having been fasted overnight (one group: 4 rats) were used. To each rat was orally administered a suspension (2 ml) of a test compound in a 0.5% carboxymethyl cellulose solution with a stomach sonde (dose of test compound: 389.4 µmole/kg, corresponding to 100 mg/kg of calcium hopanthenate). One or two hours after the administration, the rats were killed by cutting whole carotid arteries, and the blood was collected. The blood of each rat was centrifuged (2,800 r.p.m., 15 minutes) to separate blood plasma. Concentration of hopanthenic acid in the blood plasma (0.1 ml) was measured by gas chromatography and mass spectrometry. The results are shown in Table 1.

TABLE 1

| Compound No. | Level of hopanthenic acid in the blood plasma (µg /ml) (average ± S.D.) | |
|---|---|---|
| | One hour after the administration | Two hours after the administration |
| 1 | 32.9 ± 2.7 | 16.4 ± 3.5 |
| 2 | 37.3 ± 1.8 | 21.3 ± 1.8 |
| Control | 19.4 ± 1.9 | 14.2 ± 1.6 |

[Remarks] :

Compound No. 1 : D-4-[N-Isobutyryloxy-3,3-dimethyl-2-hydroxy-n-butyryl)amino]-n-butyric acid

Compound No. 2 : D-4-[N-(4-Isovaleryloxy-3,3-dimethyl-2-hydroxy-n-butyryl)amino]-n-butyric acid

Control : Calcium D-4-[N-(2,4-dihydroxy-3,3-dimethyl-n-butyryl)amino]-n-butyrate

Experiment 2

SD-male rats, weighing about 200 g, 7 weeks age, having been fasted overnight (one group: 4 rats) were used. To each rat was orally administered a suspension (2 ml) of a test compound in a 0.5% carboxymethyl cellulose solution with a stomach sonde (dose of test compound: 389.4 µmole/kg, corresponding to 100 mg/kg of calcium hopanthenate). Two or three hours after the administration, the cerebrum was taken out and washed with physiological saline solution. To the cerebrum was added 9 times volume of water, and the mixture was homogenated with a homogenizer for one minute under ice-cooling and then centrifuged at 10,000 r.p.m. at 4°C for one hour. Concentration of hopanthenic acid in the supernatant (1 ml, corresponding to 0.1 g of the cerebrum) was measured by gas chromatography and mass spectrometry. The results are shown in Table 2. The test compounds were the same as used in Experiment 1.

TABLE 2

| Compound No. | Level of hopanthenic acid in the cerebrum (μg /g) (average ± S.D.) | |
|---|---|---|
| | Two hours after the administration | Three hours after the administration |
| 1 | 1.57 ± 0.25 | 0.63 ± 0.13 |
| 2 | 1.42 ± 0.32 | 0.56 ± 0.07 |
| Control | 0.70 ± 0.11 | 0.37 ± 0.07 |

As is clear from the above experimental results, when administered orally to rats in a dose of 389.4 μmole/kg, the compounds of the present invention: D-4-[N-(4-isobutyryloxy-3,3-dimethyl-2-hydroxy-n-butyryl]-n-butyric acid and D-4-[N-(4-isovaleryloxy-3,3-dimethyl-2-hydroxy-n-butyryl)amino]-n-butyric acid showed a high blood plasma level of hopanthenic acid such as about 1.2 to 1.9 times higher than the level in case of administering calcium hopanthenate, and also showed a high cerebrum level of hopanthenic acid such as about 1.5 to 2.2 times higher than the level in case of administering calcium hopanthenate.

Besides, the compounds (I) of the present invention have low toxicity and hence have high safety. For instance, the compounds of the present invention: D-4-[N-(4-isobutyryloxy-3,3-dimethyl-2-hydroxy-n-butyryl]-n-butyric acid and D-4-[N-(4-isovaleryloxy-3,3-dimethyl-2-hydroxy-n-butyryl)amino]-n-butyric acid showed a maximum tolerance of above 2,000 mg/kg in mice (said maximum tolerance was measured by administering the test compound to mice and observing death of mice 48 hours after the administration; the maximum tolerance meaning the dose just smaller than the dose inducing death of mice).

Thus, the compounds of the present invention are converted into hopanthenic acid in vivo when administered and show higher level of hopanthenic acid in blood and brain in comparison with calcium hopanthenate with high safety, and hence, are useful as a medicine for amelioration of cerebral metabolism and higher brain function disorder.

The present invention is illustrated by the following Examples but should not be construed to be limited thereto.

Example 1

(1) Benzyl D-4-[N-(2,4-dihydroxy-3,3-dimethyl-n-butyryl)amino]-n-butyrate (5 g) is dissolved in tetrahydrofuran (50 ml), and thereto is added pyridine (3 g). To the mixture is added dropwise with stirring a solution of isobutyryl chloride (1.8 g) in tetrahydrofuran (5 ml) at 0—5°C. The mixture is stirred at room temperature overnight and concentrated under reduced pressure. The resulting residue is dissolved in ethyl acetate, and the solution is washed with dilute hydrochloric acid, water, aqueous sodium bicarbonate solution and saturated saline solution in order. The ethyl acetate solution is dried and concentrated under reduced pressure to remove the solvent. The residue is purified by silica gel chromatography (solvent; chloroform:ethyl acetate = 4:1) to give benzyl D-4-[N-(4-isobutyryloxy-3,3-dimethyl-2-hydroxy-n-butyryl)-amino]-n-butyrate (3.6 g, 59.2%) as a colorless viscous oil. IR $\nu_{max}^{film}$ ($cm^{-1}$): 3350, 1730, 1650, Mass (m/e): 393 ($M^+$).

(2) Benzyl D-4-[N-(4-isobutyryloxy-3,3-dimethyl-2-hydroxy-n-butyryl)amino]-n-butyrate (3.4 g) is dissolved in methanol (34 ml), and thereto is added palladium black (30 mg). The mixture is subjected to catalytic reduction at room temperature under atmospheric pressure. After the reaction, the reaction mixture is filtered to remove undissolved substances, and the filtrate is concentrated under reduced pressure. The residue is recrystallized from ethyl acetate-n-hexane to give D-4-[N-(4-isobutyryloxy-3,3-dimethyl-2-hydroxy-n-butyryl)amino]-n-butyric acid (2.0 g, 76.3%) as colorless prisms. M.p. 73—75°C, IR $\nu_{max}^{nujol}$ ($cm^{-1}$): 3320, 1715, 1610, Mass (m/e): 303 ($M^+$), $[\alpha]_D^{26}$ +37.2° (c=1, ethanol).

(3) D-4-[N-(4-isobutyryloxy-3,3-dimethyl-2-hydroxy-n-butyryl)amino]-n-butyric acid (1.0 g) is dissolved in ethanol (20 ml) and thereto are added calcium hydroxide (0.15 g) and water (2 ml). The mixture is stirred at room temperature for about 40 minutes. The reaction mixture is filtered to remove undissolved substances and the filtrate is concentrated under reduced pressure. The residue is treated with n-hexane to give calcium D-4-[N-(4-isobutyryloxy-3,3-dimethyl-2-hydroxy-n-butyryl)amino]-n-butyrate (0.85 g, 80.0%) as colorless powder. $[\alpha]_D^{21}$ +28.3° (c=1, ethanol).

## Example 2

(1) In the same manner as described in Example 1 (1) using benzyl D-4-[N-(2,4-dihydroxy-3,3-dimethyl-n-butyryl)amino]-n-butyrate (5.0 g), tetrahydrofuran (55 ml), pyridine (3 g) and isovaleryl chloride (2.1 g), there is obtained benzyl D-4-[N-(4-isovaleryloxy-3,3-dimethyl-2-hydroxy-n-butyryl)amino]-n-butyrate (4.0 g, 63.5%) as a colorless viscous oil. IR $\nu_{max}^{nujol}$ ($cm^{-1}$): 3350, 1730, 1650, Mass (m/e): 407.

(2) In the same manner as described in Example 1 (2) using benzyl D-4-[N-(4-isovaleryloxy-3,3-dimethyl-2-hydroxy-n-butyryl)amino]-n-butyrate (3.8 g), methanol (38 ml) and palladium black (30 mg), there is obtained D-4-[N-(4-isovaleryloxy-3,3-dimethyl-2-hydroxy-n-butyryl)amino]-n-butyrate (2.1 g, 70.9%) as colorless prisms. M.p. 84—86°C, IR $\nu_{max}^{nujol}$ ($cm^{-1}$): 3300, 1720, 1610, Mass (m/e): 317 ($M^+$), $[\alpha]_D^{26}$ +34.1° (c=1, ethanol).

(3) In the same manner as described in Example 1 (3) using D-4-[N-(4-isovaleryloxy-3,3-dimethyl-2-hydroxy-n-butyryl)amino]-n-butyric acid (1.0 g), ethanol (20 ml), calcium hydroxide (0.13 g) and water (2 ml), there is obtained calcium D-4-[N-(4-isovaleryloxy-3,3-dimethyl-2-hydroxy-n-butyryl)amino]-n-butyrate (0.84 g, 79.2%) as a colorless powder. $[\alpha]_D^{21}$ +26.1° (c=1, ethanol).

## Example 3

(1) In the same manner as described in Example 1 (1) using benzyl D-4-[N-(2,4-dihydroxy-3,3-dimethyl-n-butyryl)amino]-n-butyrate (5.0 g), tetrahydrofuran (55 ml), pyridine (3 g) and isohexanoyl chloride (2.3 g), there is obtained benzyl D-4-[N-(4-isohexanoyloxy-3,3-dimethyl-2-hydroxy-n-butyryl)amino]-n-butyrate (4.8 g, 73.1%) as a colorless viscous oil. IR $\nu_{max}^{nujol}$ ($cm^{-1}$): 3350, 1730, 1650, Mass (m/e): 421 ($M^+$).

(2) In the same manner as described in Example 1 (2) using benzyl D-4-[N-(4-isohexanoyloxy-3,3-dimethyl-2-hydroxy-n-butyryl)amino]-n-butyrate (4.0 g), methanol (40 ml) and palladium black (40 mg), there is obtained D-4-[N-(4-isohexanoyloxy-3,3-dimethyl-2-hydroxy-n-butyryl)amino]-n-butyric acid (2.9 g, 92.2%) as a colorless viscous oil. IR $\nu_{max}^{film}$ ($cm^{-1}$): 3330, 1720, 1610, Mass (m/e): 331 ($M^+$), $[\alpha]_D^{26}$ +31.6° (c=1, ethanol).

## Example 4

(1) In the same manner as described in Example 1 (1) using benzyl D-4-[N-(2,4-dihydroxy-3,3-dimethyl-n-butyryl)amino]-n-butyrate (4.0 g), tetrahydrofuran (45 ml), pyridine (2.4 g) and pivaloyl chloride (1.8 g), there is obtained benzyl D-4-[N-(4-pivaloyloxy-3,3-dimethyl-2-hydroxy-n-butyryl)amino]-n-butyrate (3.0 g, 47.6%) as a colorless viscous oil. IR $\nu_{max}^{film}$ ($cm^{-1}$): 3350, 1730, 1650, Mass (m/e): 407 ($M^+$).

(2) In the same manner as described in Example 1 (2) using benzyl D-4-[N-(4-pivaloyloxy-3,3-dimethyl-2-hydroxy-n-butyryl)amino]-n-butyrate (2.8 g), methanol (28 ml) and palladium black (30 mg), there is obtained D-4-[N-(4-pivaloyloxy-3,3-dimethyl-2-hydroxy-n-butyryl)amino]-n-butyric acid (1.1 g, 50.4%) as colorless crystals. M.p. 79—82°C recrystallized from a mixture of ethyl acetate and n-hexane, IR $\nu_{max}^{nujol}$ ($cm^{-1}$): 3350, 1730, 1710, 1610, Mass (m/e): 317 ($M^-$).

## Example 5

(1) In the same manner as described in Example 1 (1) using benzyl D-4-[N-2,4-dihydroxy-3,3-dimethyl-n-butyryl)amino]-n-butyrate (4.0 g), tetrahydrofuran (45 ml), pyridine (2.4 g) and 3,3-dimethyl-n-butyryl chloride (2.0 g), there is obtained benzyl D-4-[N-(4-(3,3-dimethyl-n-butyryl)oxy-3,3-dimethyl-2-hydroxy-n-butyryl)amino]-n-butyrate (3.7 g, 71.0%) as a colorless sticky oily substance. IR $\nu_{max}^{film}$ ($cm^{-1}$): 3350, 1730, 1650, Mass (m/e): 421 ($M^+$).

(2) In the same manner as described in Example 1 (2) using benzyl D-4-[N-(4-(3,3-dimethyl-n-butyryl)oxy-3,3-dimethyl-2-hydroxy-n-butyryl)amino]-n-butyrate (3.5 g), methanol (35 ml) and palladium black (40 mg), there is obtained D-4-[N-(4-(3,3-dimethyl-n-butyryl)oxy-3,3-dimethyl-2-hydroxy-n-butyryl)-amino]-n-butyric acid (2.2 g, 79.7%) as colorless crystals. M.p. 102—104°C, IR $\nu_{max}^{nujol}$ ($cm^{-1}$): 3340, 1735, 1715, 1610, Mass (m/e): 331 ($M^+$), $[\alpha]_D^{26}$ +32.0° (c=1, ethanol).

## Example 6

(1) In the same manner as described in Example 1 (1) using benzyl D-4-[N-(2,4-dihydroxy-3,3-dimethyl-n-butyryl)amino]-n-butyrate (4.0 g), tetrahydrofuran (45 ml), pyridine (2.4 g) and 2-ethyl-n-butyryl chloride (2.0 g), there is obtained benzyl D-4-[N-(4-(2-ethyl-n-butyryl)oxy-3,3-dimethyl-2-hydroxy-n-butyryl)amino]-n-butyrate (2.5 g, 47.9%) as a colorless viscous oil. IR $\nu_{max}^{film}$ ($cm^{-1}$): 3350, 1730, 1650, Mass (m/e): 421 ($M^+$).

(2) In the same manner as described in Example 1 (2) using benzyl D-4-[N-(4-(2-ethyl-n-butyryl)oxy-3,3-dimethyl-2-hydroxy-n-butyryl)amino]-n-butyrate (2.4 g), methanol (24 ml) and palladium black (30 mg), there is obtained D-4-[N-(4-(2-ethyl-n-butyryl)oxy-n-butyryl)amino]-n-butyric acid (1.7 g, 90.1%) as a colorless viscous oil. IR $\nu_{max}^{film}$ ($cm^{-1}$): 3350, 1730, 1710, 1645, Mass (m/e): 331 ($M^+$), $[\alpha]_D^{19}$ +25.9° (c=1, ethanol).

(3) In the same manner as described in Example 1 (3) using D-4-[N-(4-(2-ethyl-n-butyryl)oxy-3,3-dimethyl-2-hydroxy-n-butyryl)amino]-n-butyric acid (1.0 g), ethanol (20 ml), calcium hydroxide (130 mg) and water (2 ml), there is obtained calcium D-4-[N-(4-(2-ethyl-n-butyryl)oxy-3,3-dimethyl-2-hydroxy-n-butyryl)amino]-n-butyrate (0.81 g, 76.6%) as a colorless powder. $[\alpha]_D^{21}$ +24.7° (c=1, ethanol).

Example 7

Calcium D-4-[N-(2,4-dihydroxy-3,3-dimethyl-n-butyryl)amino]-n-butyrate hemihydrate (5.2 g) is dissolved in water (20 ml), and thereto are simultaneously added dropwise with stirring 1N aqueous sodium hydroxide (40 ml) and isobutyric anhydride (6.4 g) under ice-cooling over a period of about 30 minutes, while keeping a pH of the mixture to 8—9. After the addition, the mixture is stirred at the same temperature for 40 minutes. The reaction mixture is washed with ethyl acetate, and the aqueous layer is made acidic with 10% hydrochloric acid and the extracted with ethyl acetate. The extract is washed with water, dried and then concentrated under reduced pressure. The residue is recrystallized from ethyl acetate-n-hexane to give D-4-[N-(4-isobutyryloxy-3,3-dimethyl-2-hydroxy-n-butyryl)amino]-n-butyric acid (2.3 g, 37.4%) as colorless crystals.

The physicochemical properties of this product are identical with those of the product obtained in Example 1 (2).

Example 8

In the same manner as described in Example 7 using calcium D-4-[N-(2,4-dihydroxy-3,3-dimethyl-n-butyryl)amino]-n-butyrate hemihydrate (5.2 g), isovaleric anhydride (7.4 g) and 1N aqueous sodium hydroxide (30 ml), there is obtained D-4-[N-(4-isovaleryloxy-3,3-dimethyl-2-hydroxy-n-butyryl)amino]-n-butyric acid (1.9 g, 29.6%) as colorless crystals.

The physicochemical properties of this product are identical with those of the product obtained in Example 2 (2).

Example 9

(1) Benzyl D-4-[N-(2,4-dihydroxy-3,3-dimethyl-n-butyryl)amino]-n-butyrate (4.0 g) is dissolved in tetrahydrofuran (30 ml) and thereto is added pyridine (2 ml). To the mixture is added dropwise a solution of acetyl chloride (1.2 g) in tetrahydrofuran (5 ml) under ice-cooling. The mixture is stirred at room temperature overnight and then concentrated under reduced pressure. The residue is dissolved in ethyl acetate, and the solution is washed with dilute hydrochloric acid, water, aqueous sodium bicarbonate and saline solution in order. The ethyl acetate layer is dried and concentrated under reduced pressure. The residue is purified by silica gel chromatography (solvent; chloroform:ethyl acetate = 4:1) to give benzyl D-4-[N-(4-acetoxy-3,3-dimethyl-2-hydroxy-n-butyryl)amino]-n-butyrate (2.3 g, 50.9%) as a colorless viscous oil. IR $\nu_{max}^{film}$ ($cm^{-1}$): 3350, 1730, 1650, Mass (m/e): 365 ($M^+$).

(2) Benzyl D-4-[N-(4-acetoxy-3,3-dimethyl-2-hydroxy-n-butyryl)amino]-n-butyrate (2.0 g) is dissolved in methanol (20 ml) and thereto is added palladium black (20 mg). The mixture is subjected to catalytic reduction at room temperature under atmospheric pressure. After the reaction, the reaction mixture is filtered to remove undissolved substances, and the filtrate is concentrated under reduced pressure to give D-4-[N-(4-acetoxy-3,3-dimethyl-2-hydroxy-n-butyryl)amino]-n-butyric acid (1.38 g, 91.6%) as a colorless viscous oil. IR $\nu_{max}^{film}$ ($cm^{-1}$): 3350, 1720, 1640, Mass (m/e): 275 ($M^+$), $[\alpha]_D^{20}$ +33.1° (c=1, ethanol).

Example 10

(1) In the same manner as described in Example 9 (1) using benzyl D-4-[N-(2,4-dihydroxy-3,3-dimethyl-n-butyryl)amino]-n-butyrate (4.0 g), tetrahydrofuran (35 ml), pyridine (2 ml) and propionyl chloride (1.4 g), there is obtained benzyl D-4-[N-(4-propionyloxy-3,3-dimethyl-2-hydroxy-n-butyryl)amino]-n-butyrate (2.2 g, 46.9%) as a colorless viscous oil. IR $\nu_{max}^{film}$ ($cm^{-1}$): 3350, 1730, 1650, Mass (m/e): 379 ($M^+$).

(2) In the same manner as described in Example 9 (2) using benzyl D-4-[N-(4-propionyloxy-3,3-dimethyl-2-hydroxy-n-butyryl)amino]-n-butyrate (2.0 g), methanol (20 ml) and palladium black (20 mg), there is obtained D-4-[N-(4-propionyloxy-3,3-dimethyl-2-hydroxy-n-butyryl)amino]-n-butyric acid (1.46 g, 95.7%) as a colorless viscous oil. IR $\nu_{max}^{film}$ ($cm^{-1}$): 3350, 1720, 1640, Mass (m/e): 289 ($M^+$), $[\alpha]_D^{20}$ +30.0° (c=1, ethanol).

Example 11

(1) In the same manner as described in Example 9 (1) using benzyl D-4-[N-(2,4-dihydroxy-3,3-dimethyl-n-butyryl)amino]-n-butyrate (4.0 g), tetrahydrofuran (35 ml), pyridine (2 ml) and n-butyryl chloride (1.6 g), there is obtained benzyl D-4-[N-(4-n-butyryloxy-3,3-dimethyl-2-hydroxy-n-butyryl)amino]-n-butyrate (2.9 g, 59.6%) as a colorless viscous oil. IR $\nu_{max}^{film}$ ($cm^{-1}$): 3370, 1730, 1650, Mass (m/e): 393 ($M^+$), $[\alpha]_D^{25}$ +27.6° (c=1, ethanol).

(2) In the same manner as described in Example 9 (2) using benzyl D-4-[N-(4-n-butyryloxy-3,3-dimethyl-2-hydroxy-n-butyryl)amino]-n-butyrate (2.0 g), methanol (20 ml) and palladium black (20 mg), there is obtained D-4-[N-(4-n-butyryloxy-3,3-dimethyl-2-hydroxy-n-butyryl)amino]-n-butyric acid (1.48 g, 96.0%) as a colorless viscous oil. IR $\nu_{max}^{film}$ ($cm^{-1}$): 3350, 1720, 1640, Mass (m/e): 303 ($M^+$), $[\alpha]_D^{20}$ +30.2° (c=1, ethanol).

Example 12

(1) In the same manner as described in Example 9 (1) using benzyl D-4-[N-(,4-dihydroxy-3,3-dimethyl-n-butyryl)amino]-n-butyrate (4.0 g), tetrahydrofuran (35 ml), pyridine (2 ml) and n-valerylchloride (1.8 g), there is obtained benzyl D-4-[N-(4-n-valeryloxy-3,3-dimethyl-2-hydroxy-n-butyryl)amino]-n-butyrate (3.0 g, 59.5%) as a colorless crystals. M.p. 34—35°C. IR $\nu_{max}^{nujol}$ ($cm^{-1}$): 3370, 1730, 1650, Mass (m/e): 407 ($M^+$).

(2) In the same manner as described in Example 9 (2) using benzyl D-4-[N-(4-valeryloxy-3,3-dimethyl-2-hydroxy-n-butyryl)amino]-n-butyrate (2.6 g), methanol (30 ml) and palladium black (30 mg), there is obtained D-4-[N-(4-n-valeryloxy-3,3-dimethyl-2-hydroxy-n-butyryl)amino]-n-butyric acid (1.9 g, 93.8%) as a colorless viscous oil. IR $\nu_{max}^{film}$ ($cm^{-1}$): 3350, 1720, 1640, Mass (m/e): 317 ($M^+$), $[\alpha]_D^{20}$ +31.8° (c=1, ethanol).

(3) D-4-[N-(4-Valeryloxy-3,3-dimethyl-2-hydroxy-n-butyryl)amino]-n-butyric acid (0.9 g) is dissolved in methanol (10 ml), and thereto is added L-lysine (0.41 g). The mixture is stirred at room temperature for 30 minutes. The reaction mixture is concentrated under reduced pressure to remove the solvent. The residue is treated with n-hexane, and the resulting powder is collected by filtration to give D-4-[N-(4-valeryloxy-3,3-dimethyl-2-hydroxy-n-butyryl)amino]-n-butyric acid L-lysine salt (1.13 g, 86.3%) as a colorless powder. M.p. 130—133°C, $[\alpha]_D^{20}$ +25.5° (c=1, ethanol).

(4) D-4-[N-(4-n-Valeryloxy-3,3-dimethyl-2-hydroxy-n-butyryl)amino]-n-butyric acid (1.0 g) is dissolved in methanol (10 ml), and thereto are added calcium hydroxide (0.12 g) and water (5 ml). The mixture is stirred at room temperature and then concentrated under reduced pressure. To the residue is added ethanol, and the mixture is concentrated to dryness under reduced pressure to give calcium D-4-[N-(4-n-valeryloxy-3,3-dimethyl-2-hydroxy-n-butyryl)amino]-n-butyrate (1.0 g, 94.3%) as a colorless powder.

Example 13

(1) In the same manner as described in Example 9 (1) using benzyl D-4-[N-2,4-dihydroxy-3,3-dimethyl-n-butyryl)amino]-n-butyrate (4.0 g), tetrahydrofuran (35 ml), pyridine (2 ml) and n-hexanoyl chloride (2.0 g), there is obtained benzyl D-4-[N-(4-n-hexanoyloxy-3,3-dimethyl-2-hydroxy-n-butyryl)amino]-n-butyrate (3.2 g, 61.4%) as a colorless viscous oil. IR $\nu_{max}^{film}$ ($cm^{-1}$): 3370, 1730, 1650, Mass (m/e): 421 ($M^+$).

(2) In the same manner as described in Example 9 (2) using benzyl D-4-[N-(4-n-hexanoyloxy-3,3-dimethyl-2-hydroxy-n-butyryl)amino]-n-butyrate (2.0 g), methanol (20 ml) and palladium black (20 mg), there is obtained D-4-[N-(4-hexanoyloxy-3,3-dimethyl-2-hydroxy-n-butyryl)amino]-n-butyric acid (1.35 g, 85.9%) as a colorless viscous oil. IR $\nu_{max}^{film}$ ($cm^{-1}$): 3350, 1720, 1640, Mass (m/e): 331 ($M^+$), $[\alpha]_D^{20}$ +27.6° (c=1, ethanol).

(3) In the same manner as described in Example 12 (3) using D-4-[N-(4-n-hexanoyloxy-3,3-dimethyl-2-hydroxy-n-butyryl)amino]-n-butyric acid (1.0 g), L-lysine (0.44 g) and methanol (10 ml), there is obtained D-4-[N-(4-n-hexanoyloxy-3,3-dimethyl-2-hydroxy-n-butyryl)amino]-n-butyric acid L-lysine salt (1.3 g, 90.2%) as a colorless powder. M.p. 131—134·C, $[\alpha]_D^{20}$ +25.1° (c=0.5, ethanol).

Example 14

(1) In the same manner as described in Example 9 (1) using benzyl D-4-[N-2,4-dihydroxy-3,3-dimethyl-n-butyryl)amino]-n-butyrate (4.0 g), tetrahydrofuran (35 ml), pyridine (2 ml) and n-heptanoyl chloride (2.2 g), there is obtained benzyl D-4-[N-(4-n-heptanoyloxy-3,3-dimethyl-2-hydroxy-n-butyryl)amino]-n-butyrate (2.5 g, 46.4%) as a colorless viscous oil. IR $\nu_{max}^{film}$ ($cm^{-1}$): 3370, 1730, 1650, Mass (m/e): 435 ($M^+$).

(2) In the same manner as described in Example 9 (2) using benzyl D-4-[N-(4-n-heptanoyloxy-3,3-dimethyl-2-hydroxy-n-butyryl)amino]-n-butyrate (1.8 g), methanol (20 ml) and palladium black (20 mg), there is obtained D-4-[N-(4-n-heptanoyloxy-3,3-dimethyl-2-hydroxy-n-butyryl)amino]-n-butyric acid (1.3 g, 91.1%) as a colorless viscous oil. IR $\nu_{max}^{film}$ ($cm^{-1}$): 3330, 1720, 1640, Mass (m/e): 345 ($M^+$), $[\alpha]_D^{23}$ +30.2° (c=1, ethanol).

Example 15

(1) In the same manner as described in Example 9 (1) using benzyl 4-[N-(2,4-dihydroxy-3,3-dimethyl-n-butyryl)amino]-n-butyrate (4.0 g), tetrahydrofuran (35 ml), pyridine (2 ml) and n-octanoyl chloride (2.4 g), there is obtained benzyl D-4-[N-(4-n-octanoyloxy-3,3-dimethyl-2-hydroxy-n-butyryl)amino]-n-butyrate (2.2 g, 39.6%) as a colorless viscous oil. IR $\nu_{max}^{film}$ ($cm^{-1}$): 3370, 1730, 1650, Mass (m/e): 449 ($M^+$).

(2) In the same manner as described in Example 9 (2) using benzyl D-4-[N-(4-n-octanoyloxy-3,3-dimethyl-2-hydroxy-n-butyryl)amino]-n-butyrate (1.4 g), methanol (20 ml) and palladium black (15 mg), there is obtained D-4-[N-(4-n-octanoyloxy-3,3-dimethyl-2-hydroxy-n-butyryl)amino]-n-butyric acid (1.05 g, 93.8%) as a colorless viscous oil. IR $\nu_{max}^{film}$ ($cm^{-1}$): 3340, 1710, 1640, Mass (m/e): 359 ($M^+$), $[\alpha]_D^{23}$ +27.4° (c=0.5, ethanol).

Example 16

(1) In the same manner as described in Example 9 (1) using benzyl D-4-[N-2,4-dihydroxy-3,3-dimethyl-n-butyryl)amino]-n-butyrate (4.0 g), tetrahydrofuran (35 ml), pyridine (2 ml) and n-nonanoyl chloride (2.6 g), there is obtained benzyl D-4-[N-(4-n-nonanoyloxy-3,3-dimethyl-2-hydroxy-n-butyryl)amino]-n-butyrate (3.9 g, 68.0%) as a colorless viscous oil. IR $\nu_{max}^{film}$ ($cm^{-1}$): 3370, 1735, 1650, Mass (m/e): 463 ($M^+$).

(2) In the same manner as described in Example 9 (2) using benzyl D-4-[N-(4-n-nonanoyloxy-3,3-dimethyl-2-hydroxy-n-butyryl)amino]-n-butyrate (2.0 g), methanol (20 ml) and palladium black (20 mg), there is obtained D-4-[N-(4-n-nonanoyloxy-3,3-dimethyl-2-hydroxy-n-butyryl)amino]-n-butyric acid (1.4 g, 86.9%) as a colorless viscous oil. IR $\nu_{max}^{film}$ ($cm^{-1}$): 3330, 1720, 1640, Mass (m/e): 373 ($M^+$), $[\alpha]_D^{18}$ +26.2° (c=1, ethanol).

(3) In the same manner as described in Example 12 (3) using D-4-[N-(4-n-nonanoyloxy-3,3-dimethyl-2-hydroxy-n-butyryl)amino]-n-butyric acid (0.9 g), methanol (10 ml) and L-lysine (0.35 g), there is obtained D-4-[N-(4-n-nonanoyloxy-3,3-dimethyl-2-hydroxy-n-butyryl)amino]-n-butyric acid L-lysine salt (1.15 g, 91.8%) as a colorless powder. M.p. 158—161°C, $[\alpha]_D^{18}$ +22.9° (c=0.5, ethanol).

Reference Example

(1) Calcium D-4-[N-(2,4-dihydroxy-3,3-dimethyl-n-butyryl)amino]-n-butyrate hemihydrate (100 g) is dissolved in water (500 ml), and the solution is passed through a column of an acidic type ion-exchange resin, followed by washing the column with water. The effluent and washing liquid are combined and thereto is added dicyclohexylamine (71 g). The mixture is washed with ether, and the aqueous layer is concentrated under reduced pressure. The residue is crystallized from ethyl acetate. The precipitates crystals are separated by filtration and washed with ether to give D-4-[N-(2,4-dihydroxy-3,3-dimethyl-n-butyryl)amino]-n-butyric acid dicyclohexylamine salt (122 g, 75.6%) as colorless crystals.

(2) D-4-[N-2,4-Dihydroxy-3,3-dimethyl-n-butyryl)amino]-n-butyric acid dicyclohexylamine salt (121 g) is mixed with benzyl bromide (50 g) and dimethylformamide (700 ml), and the mixture is stirred at about 60°C for 6 hours. The reaction mixture is filtered to remove undissolved substances and the filtrate is concentrated under reduced pressure. The residue is dissolved in ethyl acetate, and the solution is washed with dilute sulfuric acid, saline solution, aqueous sodium bicarbonate and saline solution in this order. The ethyl acetate layer is dried and evaporated to dryness under reduced pressure to give benzyl D-4-[N-(2,4-dihydroxy-3,3-dimethyl-n-butyryl)amino]-n-butyrate (66 g, 69.9%) as a pale yellow oil. IR $\nu_{max}^{film}$ ($cm^{-1}$): 3350, 1725, 1650, Mass (m/e): 323, $[\alpha]_D^{20}$ +32.0° (c=1, ethanol).

## Claims

1. A 4-aminobutyric acid derivative of the formula:

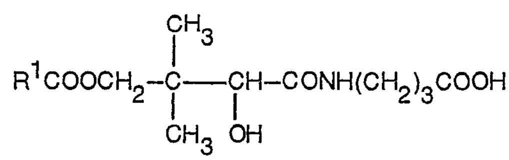

(I)

wherein $R^1$ is a straight or branched alkyl group having 1 to 8 carbon atoms, or a pharmaceutically acceptable salt thereof.

2. The compound according to claim 1, wherein $R^1$ is a straight alkyl group having 1 to 8 carbon atoms.

3. The compound according to claim 1, wherein $R^1$ is a branched alkyl group having 1 to 8 carbon atoms.

4. The compound according to claim 3, wherein $R^1$ is a member selected from the group consisting of isopropyl, isobutyl, isovaleryl, tert-butyl, 2,2-dimethylpropyl and 1-ethylpropyl.

5. The compound according to claim 4, which is D-4-[N-(4-isobutyryloxy-3,3-dimethyl-2-hydroxy-n-butyryl)amino]-n-butyric acid or a pharmaceutically acceptable salt thereof.

6. The compound according to claim 4, which is D-4-[N-(4-isovaleryloxy-3,3-dimethyl-2-hydroxy-n-butyryl)amino]-n-butyric acid or a pharmaceutically acceptable salt thereof.

7. A process for preparing a 4-aminobutyric acid derivative of the formula:

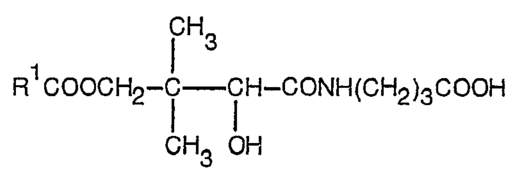

(I)

wherein $R^1$ is a straight or branched alkyl group having 1 to 8 carbon atoms, or a pharmaceutically acceptable salt thereof, which comprises subjecting a 4-aminobutyric acid derivative of the formula:

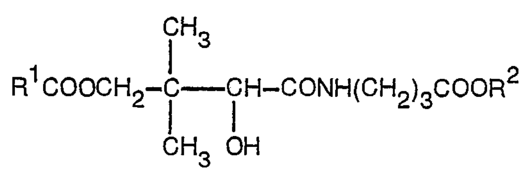

(II)

wherein $R^1$ is as defined above, and $R^2$ is a group removal by catalytic reduction, to catalytic reduction, and optionally converting the product to a pharmaceutically acceptable salt thereof.

8. A process for preparing a 4-aminobutyric acid derivative of the formula:

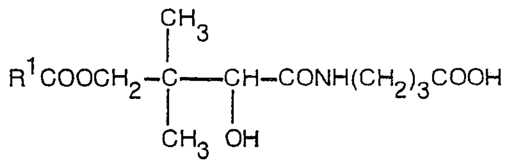

(I)

wherein $R^1$ is a straight or branched alkyl group having 1 to 8 carbon atoms, or a pharmaceutically acceptable salt thereof, which comprises reacting a 4-aminobutyric acid derivative of the formula:

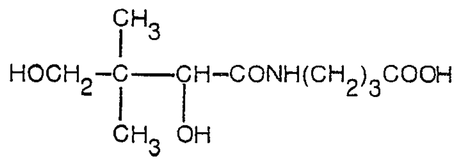

(III)

with a reactive derivative of a carboxylic acid of the formula:

$$R^1COOH \quad (IV)$$

wherein $R^1$ is as defined above, and optionally converting the product into a pharmaceutically acceptable salt thereof.

9. A pharmaceutical composition comprising an effective amount of a 4-aminobutyric acid derivative of the formula:

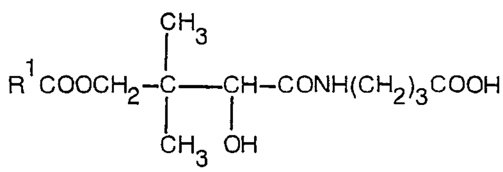

wherein $R^1$ is a straight or branched alkyl group having 1 to 8 carbon atoms, or a pharmaceutically acceptable salt thereof in admixture with a conventional pharmaceutically acceptable carrier or diluent.

**Patentansprüche**

1. Ein 4-Aminobuttersäurederivat der Formel:

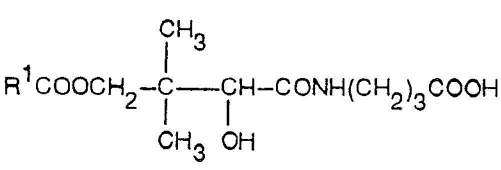

(I)

oder ein pharmazeutisch annehmbares Salz davon, worin $R^1$ eine gradkettige oder verzweigte Alkylgruppe mit 1 bis 8 Kohlenstoffatomen ist.

2. Verbindung gemäß Anspruch 1, worin $R^1$ eine gradkettige Alkylgruppe mit 1 bis 8 Kohlenstoffatomen ist.

3. Verbindung gemäß Anspruch 1, worin $R^1$ eine verzweigte Alkylgruppe mit 1 bis 8 Kohlenstoffatomen ist.

4. Verbindung gemäß Anspruch 3, worin $R^1$ ein Glied, ausgewählt aus der Gruppe bestehend aus Isopropyl, Isobutyl, Isovaleryl, Tertiärbutyl, 2,2-Dimethylpropyl und 1-Ethylpropyl, ist.

5. Verbindung gemäß Anspruch 4, nämlich D-4-[N-(4-Isobutyryloxy-3,3-dimethyl-2-hydroxy-n-butyryl)-amino]-n-Buttersäure oder ein pharmazeutisch annehmbares Salz davon.

6. Verbindung gemäß Anspruch 4, nämlich D-4-[N-(4-Isovaleryloxy-3,3-dimethyl-2-hydroxy-n-butyryl)-amino]-n-Buttersäure oder ein pharmazeutisch annehmbares Salz davon.

7. Verfahren zur Herstellung eines 4-Aminobuttersäurederivats der Formel:

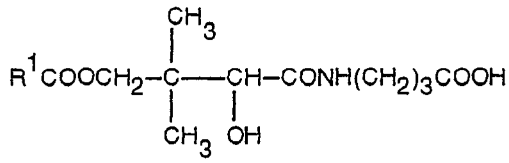

(I)

worin $R^1$ eine gradkettige oder verzweigte Alkylgruppe mit 1 bis 8 Kohlenstoffatomen bedeutet, oder eines pharmazeutisch annehmbaren Salzes davon, dadurch gekennzeichnet, daß man ein 4-Aminobuttersäurederivat der Formel:

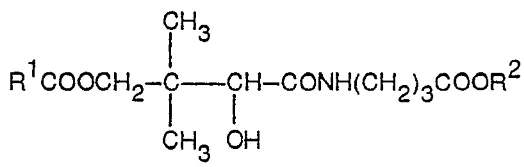

(II)

worin $R^1$ die vorher angegebene Bedeutung hat und $R^2$ eine Gruppe ist, die durch katalytische Reduktion entfernt werden kann, einer katalytischen Reduktion unterwirft und gewünschtenfalls das Produkt in ein pharmazeutisch annehmbares Salz davon überführt.

8. Verfahren zur Herstellung eines 4-Aminobuttersäurederivats der Formel:

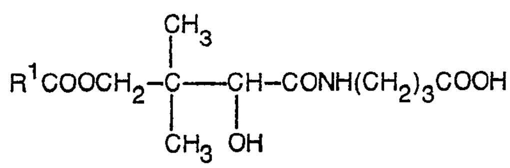

(I)

worin $R^1$ eine gradkettige oder verzweigte Alkylgruppe mit 1 bis 8 Kohlenstoffatomen ist oder eines pharmazeutisch annehmbaren Salzes davon, dadurch gekennzeichnet, daß man ein 4-Aminobuttersäurederivat der Formel:

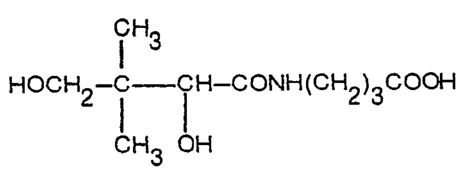

(III)

mit einem reaktiven Derivat einer Carbonsäure der Formel:

$$R^1COOH \quad (IV)$$

worin $R^1$ die vorher angegebene Bedeutung hat, umsetzt und gewünschtenfalls das Produkt in einpharmazeutisch annehmbares Salz davon überführt.

9. Pharmazeutische Zusammensetzung, umfassend eine wirksame Menge eines 4-Aminobuttersäurederivats der Formel:

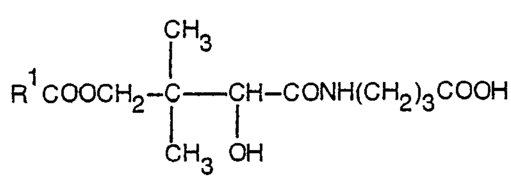

worin $R^1$ eine gradkettige oder verzweigte Alkylgruppe mit 1 bis 8 Kohlenstoffatomen bedeutet, oder ein pharmazeutisch annehmbares Salz davon, in Mischung mit einem üblichen pharmazeutisch annehmbaren Träger oder Verdünnungsmittel.

**Revendications**

1. Un dérivé d'acide 4-aminobutyrique de formule:

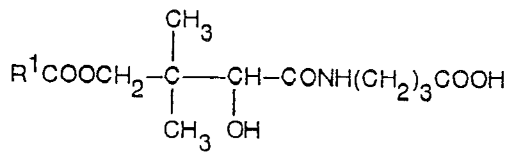

(I)

dans laquelle $R^1$ est un groupe alkyle droit ou ramifié ayant 1 à 8 atomes de carbone ou un sel convenant en pharmacie correspondant.

2. Le composé selon la revendication 1 dans lequel $R^1$ est un groupe alkyle droit ayant 1 à 8 atomes de carbone.

3. Le composé selon la revendication 1 dans lequel $R^1$ est un groupe alkyle ramifié ayant 1 à 8 atomes de carbone.

4. Le composé selon la revendication 3 dans lequel $R^1$ est un composant du groupe constitué par isopropyle, isobutyle isovaléryle, tert-butyl, 2,2-diméthylpropyle et l-éthylpropyle.

5. Le composé selon la revendication 4 qui est l'acide D-4-[4-(4-isobutyryloxy-3,3-diméthyl-2-hydroxy-n-butyryl)amino]-n-butyrique ou un de ses sels convenant en pharmacie.

6. Le composé selon la revendication 4 qui est l'acide D-4-[N-(4-isovaléryloxy-3,3-diméthyl-2-hydroxy-n-butyryl)amino]-n-butyrique ou un de ses sels convenant en pharmacie.

7. Un procédé pour préparer un dérivé d'acide 4-aminobutyrique de formule:

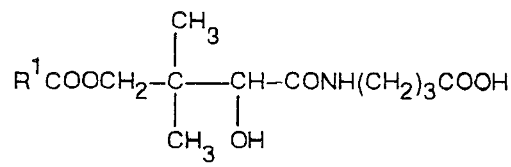

(I)

dans laquelle $R^1$ est un groupe alkyle droit ou ramifié ayant 1 à 8 atomes de carbone ou un sel convenant en pharmacie correspondant qui consiste à soumettre un dérivé d'acide 4-aminobutyrique de formule:

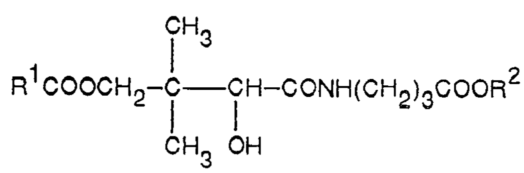

(II)

dans laquelle $R^1$ est comme défini ci-dessus et $R^2$ est un groupe éliminable par réduction catalytique, à une réduction catalytique et éventuellement transformer le produit en un de ses sels convenant en pharmacie.

8. Un procédé pour préparer un dérivé d'acide 4-aminobutyrique de formule:

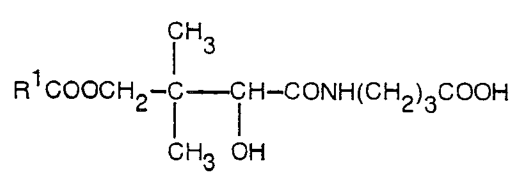

(I)

dans laquelle $R^1$ est un groupe alkyle droit ou ramifié ayant 1 à 8 atomes de carbone ou un sel convenant en pharmacie de celui-ci qui comprend la réaction d'un dérivé d'acide 4-aminobutyrique de formule:

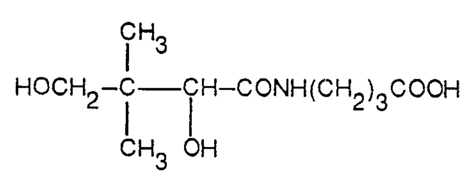

(III)

avec un dérivé réactif d'un acide carboxylique de formule:

$$R^1COOH \qquad (IV)$$

dans laquelle $R^1$ est comme défini ci-dessus et éventuellement la conversion du produit en un de ses sels convenant en pharmacie.

9. Une composition pharmaceutique comprenant une quantité efficace d'un dérivé d'acide 4-aminobutyrique de formule:

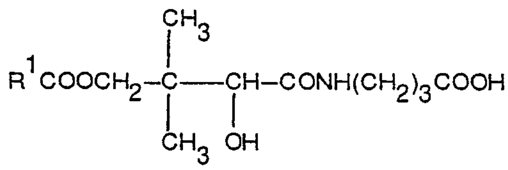

dans laquelle $R^1$ est un groupe alkyle droit ou ramifié ayant 1 à 8 atomes de carbone ou un sel convenant en pharmacie de celui-ci en mélange avec un support ou diluant classiques convenant en pharmacie.